# EUROPEAN PATENT SPECIFICATION

(11) **EP 1 163 025 B1**
(45) Date of publication and mention of the grant of the patent: **28.12.2005**
(21) Application number: 00916386.6
(22) Date of filing: 16.03.2000
(51) Int. Cl.: A61N 1/32

(54) **SWEAT CONTROL SYSTEM**
SYSTEM ZUR SCHWEISSREGULIERUNG
SYSTEME DE REGULATION DE LA TRANSPIRATION

(30) Priority: 17.03.1999 US 271932; 17.03.1999 US 271007
(43) Date of publication of application: 19.12.2001
(73) Proprietor: Tapper, Robert, Los Angeles California 90025 (US)
(72) Inventor: Tapper, Robert, Los Angeles California 90025 (US)
(74) Representative: Ebner von Eschenbach, Jennifer
(86) International application number: PCT/US2000/006860
(87) International publication number: WO 2000/054834

(56) References cited:
- DE-A- 4 137 960
- GB-A- 2 030 453
- US-A- 4 325 367
- US-A- 5 008 111
- US-A- 5 224 927

## Description

### BACKGROUND OF THE INVENTION

This invention relates generally to a hyperhidrosis treatment device, and more particularly, to an apparatus for conveniently and quickly providing enhanced iontophoretic application of antiperspirant chemicals to regions of the human body in a simple and economic manner.

Treatment of excess sweating is commonly done in one of two ways. For individuals with a mild case of sweating, effective treatment may be had through the application of chemical antiperspirants. For those inflicted with unsightly excess sweating, iontophoretic treatment may be necessary. Iontophoretic treatment involves the electrical introduction of ions into the skin to block the sweat duct.

An iontophoretic device for the treatment of hands, palms and axilla is disclosed in U.S. Patent No. 4,325,367. In this device a support structure houses a pair of aluminum alloy electrodes in generally close proximity to one another as well as a source of electrical power. The electrodes are arranged so that, for example, the palm of a hand can be placed on the device and simultaneously contact both electrodes. A moisture absorbing pad is interposed between each of the electrodes and the skin of the user. In operation, the pads are moistened with water and the user places his hand on the pads. Current is applied from the electrodes, through the pads, to the user, thereby providing iontophoretic treatment.

Another iontophoretic device is disclosed in U.S. Patent No. 5,224,927. In this device, the electrical current applied between a pair of electrodes is periodically reversed at very low frequencies to mitigate tissue damage. Again, a moisture absorbing pad is interposed between each of the electrodes and the skin of the user. In operation, the treatment site may be prepared using an appropriate ionic surfactant such as an amphoteric or a cationic surfactant and electrical current in the form of a low frequency AC signal is applied to the treatment area through either water moistened pads or ionic-surfactant moistened pads.

In the well known DRIONIC compact iontophoretic device, which employs various aspects of both U.S. Patents Nos. 4,325,367 and 5,224,927, long term treatment of severe cases of hyperhidrosis is provided through a series of iontophoretic treatments. The mean-average treatment time to effectively stop sweat is approximately seven hours. The treatment regimen calls for a series of approximately half-hour individual treatment sessions spread over the course of several days. The actual length of a session and time between sessions depends on the user's tolerance to electric current. During each treatment, metal ions, *e.g.*, aluminum ions from the electrdoes, are driven deep into the eccrine sweat pores to stop sweat. The cumulative effect of the series of treatments may stop sweat for up to six weeks. Moreover, the DRIONIC device relies on amplified voltages of 60 volts and safety control circuits.

While the DRIONIC device is intended for the extremely heavy sweater, the use of such a formidable and time consuming device may be an undesirable treatment for people suffering from only a mild case of excess sweating. Most of these individuals will chose the daily use of a wide variety of well-known over-the-counter topical antiperspirants to control heavy sweating. However, a problem associated with over-the-counter topical antiperspirants is the lack of sufficient penetration into the skin of the eccrine-pore-blocking antiperspirant chemical. Thus these topical antiperspirants have limited efficacy. It has been reported that over-the-counter topical antiperspirants are effective for only about fifty percent of those who use them. The those other fifty percent receive little or no benefit from such antiperspirants. In those people who enjoy some benefit from store bought antiperspirants, this efficacy is only between twenty and forty percent.

Hence, there has been a long existing need in the art for a system capable of administering deep-penetrating iontophoretic application of antiperspirant chemicals to the human body in a short period of time. There also exists a need for such a device that operates at a low power rating and thus relatively independent of a user's tolerance for electrical current. There further exists a need for such a device that is conducive to daily use. The present invention fulfils all of these needs and others.

### SUMMARY OF THE INVENTION

Briefly, and in general terms, the present invention is defined in claim 1 and is directed to improvements in the treatment of excess sweating and hyperhidrosis through the use of an iontophoresis administration of antiperspirant chemicals into the human body.

In a first aspect, the present invention embodies a sweat treatment device for effecting iontophoresis at a specified region of tissue. The device includes a source of electric current, a controller and a pair of electrodes. The electrodes are mounted in generally close proximity to one another and are separated by an insulating member. The electrodes carry an antiperspirant element and are responsive to the source of electrical current through the controller. One of the electrodes is connected to the source of electrical current and is arranged to act primarily as a cathode. The other electrode is connected to the source of electrical current and is arranged to act primarily as an anode. The device further includes a pair of pads. Each of the pads is positioned in adjacent contact with one of the electrodes and carries sodium salicylate. The electrodes are sized and arranged so that the tissue to be treated can extend across the insulating member and simultaneously contact both of the pads.

By carrying an appropriate salt of salicylic acid, such as sodium salicylate, the pads provide for enhanced permeability of the tissue being treated. The sodium salicylate increases the permeability of the tissue and facilitates electrical driving of the ions contained in the antiperspirant elements carried by the electrodes deeper into the tissue to precipitate the skin protein and stop sweat. Thus the device provides increase efficacy. The salicylic salt and antiperspirant chemicals would typically be replaceable.

In a more detailed aspect of the invention, the pads further carry an aluminum-based antiperspirant. In another facet, the pad antiperspirant consists of one of either aluminum-chlorohydrate or aluminum-zirconium. In yet another aspect, the electrodes are formed of sheet stock metal having an irregular, nonsmooth, surface. This nonsmooth surface creates a greater overall surface area on the electrode thereby increasing the number of antiperspirant ions available for infusion by ionthophoresis. In still another facet, the sheet stock metal consists of aluminum, aluminum alloy, magnesium or magnesium alloy. In yet another aspect, the electrodes comprise sandblasted sheet stock metal. On other aspects the electrodes comprise powered metal formed on the sheet stock metal and the electrodes comprise aluminum oxide.

In another facet, the invention is related to an enhanced device for applying iontophoresis treatment to a selected region of a biological subject. The device includes first and second electrodes mounted in generally close proximity to one another and separated by an insulating member. The device also includes a pair of pads, each positioned in adjacent contact with one of the electrodes. Each of the pads carry an antiperspirant. The electrodes are sized and arranged so that the region to be treated can extend across the insulating member and simultaneously contact both of the pads. The device further includes an electrical energy source for conducting an electrical current through the region in a first direction from the first electrode to the second electrode; and a controller for intermittently reversing, at a relatively low frequency which prevents skin damage, between approximately 20 times per second and approximately once every three minutes, the polarity of the electrodes to cause the electrical current to flow in a second direction opposite to the first direction.

By including antiperspirant in the pads and by reversing the polarity of the electrodes to reverse the direction of current flow through the region of treatment, the device provides for a greater infusion of antiperspirant ions into the region in a set amount of time. The reason for this is that if a positively charged antiperspirant is carried by a pad when the positive half of the AC signal is driving the electrode associated with the pad, then the positive component of the antiperspirant is repelled and driven into the skin.

In more detailed facets of the invention, the antiperspirant is aluminum based. In another aspect, the antiperspirant consists of one of either aluminum-chlorohydrate or aluminum-zirconium. In still another facet, the percentage of aluminum-chlorohydrate or aluminum-zirconium contained within the antiperspirant is greater than 2%. In yet another facet, the pad further carries sodium salicylate. In a further aspect, the antiperspirant comprises an anticholinergic.

In a third aspect, the invention is related to an applicator for providing the chemical materials necessary to affect iontophoresis treatment to a selected region of a biological subject. The applicator is responsive to an AC waveform provided by a generator. The applicator includes first and second electrodes mounted in generally close proximity to one another. The electrodes are separated by an insulating member and are responsive to the AC waveform. The applicator also includes a pair of pads. Each of the pads is positioned in adjacent contact with one of the electrodes and carries an antiperspirant. The electrodes are sized and arranged so that the region to be treated can extend across the insulating member and simultaneously contact both of the pads.

In more detailed facets of the invention, the first and second electrodes are responsive to the AC waveform such that the electrodes are of opposite polarities. In another aspect, the pads comprise a material containing the antiperspirant. In a still another facet, the pads are formed of a fiber and the antiperspirant is saturated into the pads. In other aspects, the antiperspirant comprises aluminum based chemicals and the antiperspirant comprises an anticholinergic.

In a fourth facet, the invention is related to a cosmetic method of iontophoretic infusion of antiperspirant substances into a biological subject to reduce unsightly sweating. The method comprises the step of locating a pair of electrically conductive electrodes adjacent a surface of the subject to be treated. The method also includes the steps of placing at least one antiperpirant substance and a suitable salt of salicylic acid, such as sodium salicylate, between at least one of the electrodes and the surface of the subject to be treated, and conducting an electrical current through the surface of the subject for a set duration of time. The current passes through the surface in a first direction from a first of the electrodes to a second of the electrodes on the subject. The method further includes the step of periodically and regularly reversing, at a relatively low frequency, between approximately 20 times per second and approximately once every three minutes, the polarity of the electrodes to cause the electrical current to flow in a second direction opposite to the first direction.

In a more detailed aspect of the invention, the set time duration for treatment is between 10 and 20 seconds and the low frequency is such that the polarity of the electrodes is reversed at least once during the set time duration.

These and other objects, aspects and advantages of the present invention will become apparent from the following more detailed description, when taken in conjunction with the accompanying drawings which illustrate, by way of example, the preferred embodiments of the invention.

### BRIEF DESCRIPTION OF THE DRAWINGS

FIGURE 1 illustrates an iontophoretic treatment device including a generator and an applicator constructed in accordance with the invention, and shown positioned in the axilla area of a human subject;
FIG. 2 is a plan view of a presently preferred embodiment of the iontophoretic device of FIG. 1, with a side cover of the generator being removed and portions of the applicator being broken away to illustrate internal structure;
FIG. 3 is a perspective view of the iontophoretic device of FIG. 1, showing the applicator being separated from the generator;
FIG. 4 is a top view of the generator;
FIG. 5 is a top view of the applicator;
FIG. 6 is a flow chart illustrating an iontophoretic process;
FIG. 7 is a general block diagram of the iontophoretic device of FIGS 1 and 2;
FIG. 8 is a schematic block diagram, including waveforms, of a iontophoretic device; and
FIG. 9 is a plan view of another presently preferred embodiment of an iontophoretic device, with a side cover of the generator being removed and portions of the applicator being broken away to illustrate internal structure.

### DETAILED DESCRIPTION OF THE PREFERRED EMBODIMENTS

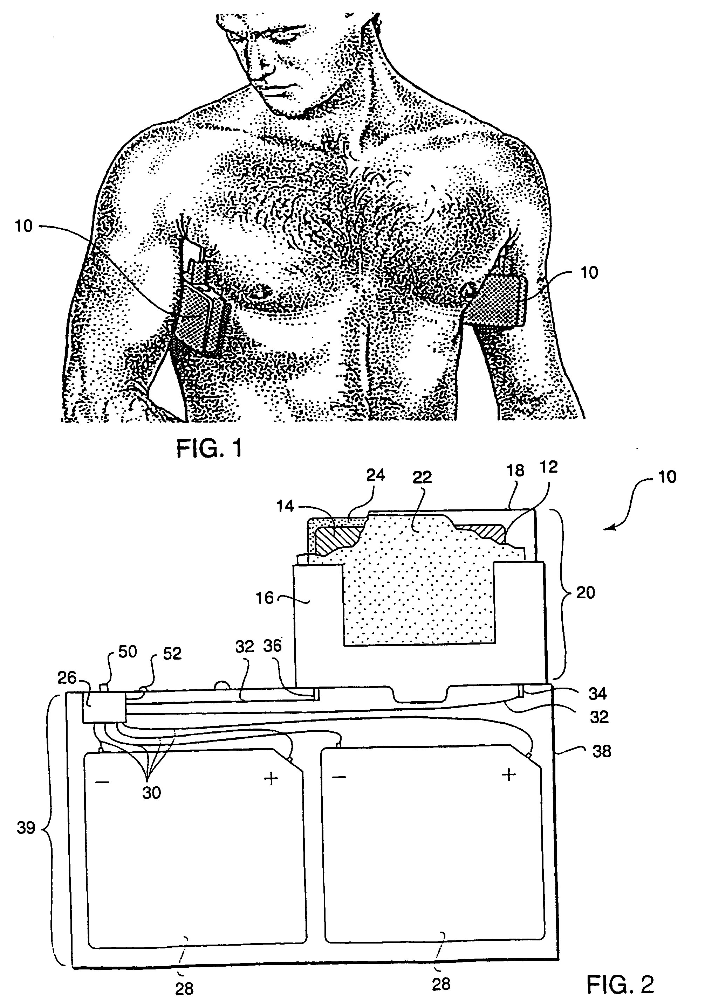

Referring now to the drawings, and more particularly to FIG.1, there is shown an iontophoretic treatment device 10, of relatively simple, economical, reliable and compact construction, embodying features of the present invention. The iontophoretic treatment device 10 is shown in use in the axilla area of a suitable biological subject so that the device contacts the skin of the subject for appropriate administration of antiperspirant chemicals by iontophoretic delivery.

While the iontophoretic device 10 is shown in a presently preferred self-contained embodiment, it will be appreciated by those of ordinary skill in the art that a larger structural and/or physical packaging unit (not shown) may be utilized, including a terminal electrode applicator for contact with the skin, and also embodying various features of the present invention.

With reference to FIGS. 2 through 5, in accordance with the present invention, an iontophoretic treatment device 10 is provided which includes an applicator 20 and a generator 39. The applicator 20 includes a pair of metallic electrodes 12, 14 mounted on a retainer 16. While the electrodes 12, 14 may be made of any metal, in a preferred embodiment they are formed of aluminum or aluminum alloy sheet stock. In another embodiment the electrodes are formed of magnesium or magnesium alloy sheet stock. In a preferred embodiment, the surface of the electrodes 12, 14 are treated to create more surface area such that more aluminum ions are available to inhibit sweat. This may be done, for example, by sandblasting the electrode 12, 14 surface or making the electrodes from powered metal. Aluminum oxide may also be added to the electrodes 12, 14. The electrodes may also be replaceable as a disposable unit.

The electrodes 12, 14 are in generally close proximity to one another and lie in generally parallel planes. The electrodes 12, 14 are separated by a relatively narrow insulating member 18. To prevent inadvertent short circuiting of the device 10 when in use, the insulating member 18 is of sufficient height to separate the pads 22, 24. Moreover, a retainer 16 is configured to ensure that no moisture from the pads 22, 24 can flow across the insulating member 18 and cause a short circuit. With this arrangement, the iontophoretic treatment device 10 can be positioned in the axilla area and held in place during treatment by the user simply lowering his arm. This arrangement has a significant advantage over prior devices in that the user's hands are relatively free during treatment, making this device particularly convenient to use.

The applicator 20 further includes a pair of moisture absorbing pads 22, 24. The pads 22, 24 are interposed between skin of the region being treated and the electrodes 12,14 to ensure adequate electrical contact with the treatment region being treated and to distribute that electrical contact over a greater area of the region. While the pads 22, 24 can be of any suitable porous synthetic or natural fiber material, it has been found that a polyester material is preferred. Polyester electrode pads soak up water much more readily than do pads made of wool felt. In addition, polyester pads do not exhibit the tendency to shrink, as do wool pads. Moreover, polyester pads are much more economical to supply, and do not support bacterial life as readily as wool felt pads.

As further explained hereinafter, the pads 22, 24 carry antiperspirant chemicals which, in combination with the aluminum electrodes, provide the ions to penetrate the skin to effectively block the sweat ducts. By "carry" it is meant that the pads are either 1) formed of a material containing the subject chemicals, 2) that the pads are formed of an absorbent material, such as felt, and are pre-soaked with the subject chemicals or 3) a combination of 1) and 2). In a preferred embodiment, the pads carry aluminum chlorohydrate or aluminum-zirconium. In another preferred embodiment, the pads carry an anticholinergic. In addition to the antiperspirant chemicals, the pads may also carry an appropriate salt of salicylic acid, such as sodium salicylate, to enhance aluminum ion skin penetration or permeability and hence efficacy of the device. Sodium salicylate has also been shown to have healing properties.

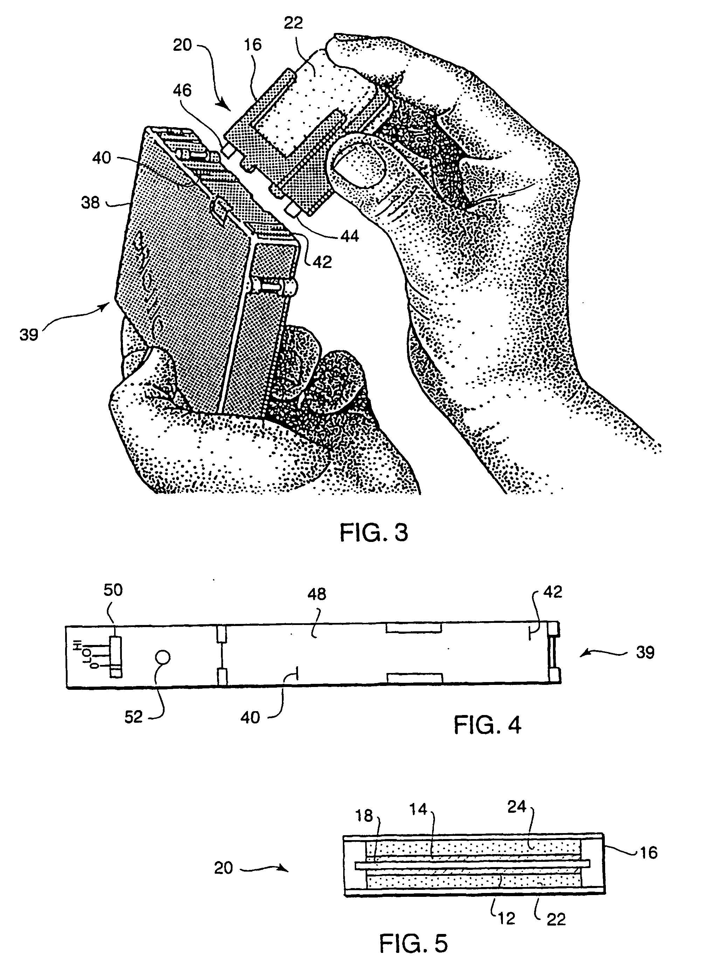

The generator 39 portion of the iontophoretic treatment device 10 includes an electronics package comprising an AC current generating chip 26, a battery power supply 28, electrical leads 30, 32, and a pair of electrical contacts 34, 36. The electronics package is contained within a housing 38. The combination of the electronics packaging and the housing 38 form the generator 39. The housing 38 includes two lead slots 40, 42 (FIGS 3 and 4) which provide access to the electrical contacts 34, 36 (FIG. 2). The retainer 16 portion of the applicator includes two electrical leads 44, 46 (FIG. 3) that snap into the lead slots 40, 42 on the housing 38 and into the electrical contacts 34, 36 (FIG. 2) and connect the battery supply 28 to the electrodes 12, 14. The teachings of U.S. Patent No. 5,224,927 are specifically incorporated herein.

The electronics package may also include an electrical slide switch 50. The switch projects through an upper plastic cover plate 48 of the housing 38. The switch 50 is electrically connected in the housing 38 to the AC generating-chip 26. The switch 50 may be selectively moved between a "0" (off) position, to either a "LO" (low current or lower rate of ion delivery) or "HI" (high current or higher rate of ion delivery) switch positions.

The function of the switch 50 in FIG. 4 is as follows:
1) The "0" position keeps the device from functioning.
2) The "LO" treatment position infuses aluminum ions at the lowest current level at a continuous, controlled rate.
3) The "HI" treatment position infuses aluminum ions at a current level typically twice as high as the "LO" setting.

A second switch (not shown), similar to the slide switch 50, may also be provided to selectively vary the frequency of the low-frequency AC duty-cycle of the iontophoretic treatment device 10. The low-frequency AC duty-cycle operation of the device is explained below.

An LED test indicator 52 extends from the electronics package within the housing 38 through an appropriate opening in the cover plate 48, and is observable from the top of the iontophoretic treatment device 10 to confirm proper electrical operation of the system for the user. The electronics package may also include a buzzer (not shown) that is connected to a timer in the AC generating chip 26. The timer may be set to sound the buzzer at a time interval, *e.g*., 10 seconds, in order to provide an indication to the user as to the cumulative treatment time.

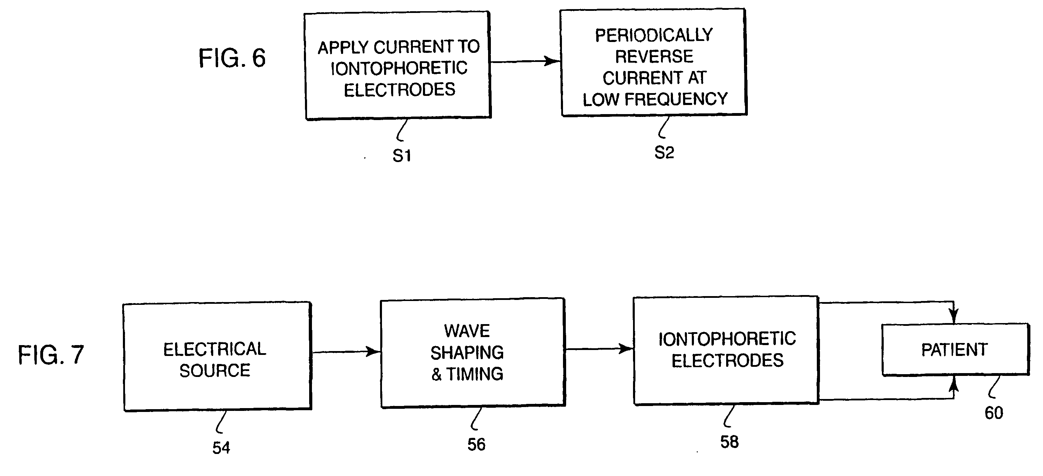

Referring now to FIG. 6, the low-frequency AC duty-cycle process which facilitates the numerous advantages of the present invention is broadly illustrated and defined. In this regard, at step S1, the process calls for applying electrical current to a pair of iontophoretic electrodes of opposite polarity, such as the electrodes 12, 14 in the iontophoretic-treatment device 10. In step S2, the electrical polarity and, therefore, the direction of the electrical current flowing between the electrodes 12,14 and through the patient is periodically reversed, twice per AC cycle. This reversal of current occurs at low frequencies in the substantially critical range of approximately 10 Hz to once every three minutes, or a low frequency limit of approximately 0.0027 Hz, to achieve the advantages previously and subsequently described herein in connection with the practice of the present invention.

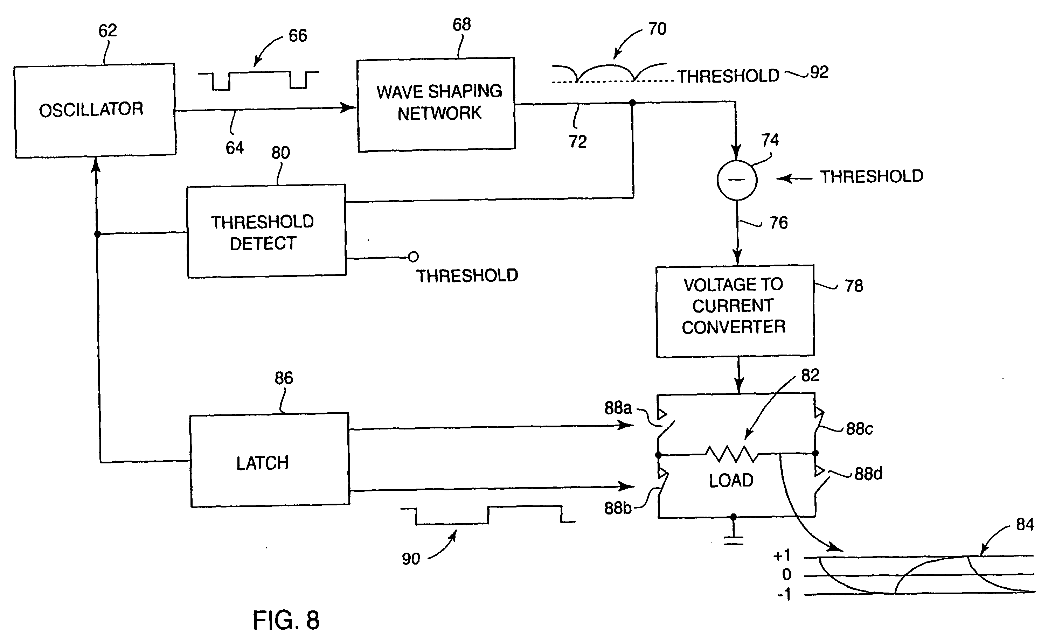

FIG. 7 is a basic block diagram illustrating the electronics package contained within the housing 38, wherein an electrical source 54 is directed to appropriate wave shaping and timing circuitry 56 for generating the aforedescribed low-frequency AC duty-cycle which is then directed as electrical current to iontophoretic electrodes 58 to infuse aluminum ions into the skin, *i.e.,* the electrical load in the system. It is apparent that the various electrical subsystems indicated in FIGS. 6 and 7 can be implemented readily by those of ordinary skill in the art without the exercise of inventive skill. For example, the system illustrated in FIG. 7 may be implemented, in a presently preferred embodiment of the invention, by the more detailed system shown in FIG. 8.

Referring now to FIG. 8, there is shown a presently preferred embodiment of an overall system for providing a regulated and periodically reversible electrical current into a variable load resistance, *i.e.,* the patient. In the system, the electrical current reverses polarity and direction of flow periodically at a very low frequency. A smooth transition without discontinuity in slope is made between polarities, thus avoiding a shock sensation to the patient when reversing the electrical current. The magnitude and duty cycle of the positive and negative currents are substantially the same. The system utilizes a conventional DC power supply.

The timing of current reversals is determined by an oscillator 62, which produces at its output 64 sharp transitions between two levels, as illustrated by the waveform 66. The electrical output 64 is applied to a wave shaping network 68 to produce gradual electrical transitions, as shown by the output waveform 70 available on line 72. The electrical output of the oscillator 62, and thus the sense of the smoothed waveform, is reversed when the waveform crosses a predetermined threshold 92 determined at junction 74 under the control of a threshold detection subsystem 80. The voltage waveform 70, less the threshold 92, is applied over line 76 to a suitable voltage-to-current converter subsystem 78.

The polarity of the electrical current through a floating load 82, *e*.*g*., the patient, reverses at the threshold crossing time, when the instantaneous electrical load current is zero, as illustrated by the waveform 84. A latch subsystem 86 controls a plurality of switches 88a-88d, as shown by the waveform 90, to maintain this polarity until the next threshold crossing. This produces smooth transitions between electrical current levels that are by design, substantially equal in magnitude but opposite in sign. The relatively slow rise and decay evident from leading and trailing edges of the waveform 84 provides the desirable electrical ramping up and down of each half cycle to minimize shock sensations.

The electronics package may provide for a fixed current reversal frequency. If desired, the electrical system may be modified, in a manner well known to those of ordinary skill in the art, to automatically vary the signal frequency periodically. One example of specific electrical circuitry, suitable for implementing the system shown in FIG. 8, is set forth in Appendix A attached hereto and which is specifically incorporated by reference herein.

In operation, the pads 22, 24 are soaked with water to activate the chemicals carried by the pad. The pads 22, 24 are then placed in the retainer 16, one adjacent each of the electrodes 12, 14. The retainer 16 is then placed in the axilla area. The switch 24 is moved from the "0" position to either the "LO" or "HI" position as prescribed below. Electrical current is thus applied to the electrodes 12, 14 by the generator 39 to direct ions into the eccrine duct for sweat control. The aluminun ions from the electrodes 12, 14 and from the chemicals carried by the pads 22, 24 precipitate the skin protein and plug the sweat pores.

In one embodiment of the invention, for treatment of mild cases of excess sweating, the treatment regimen comprises one 10 to 20 second session. The system is optimally operated such that at least one cycle occurs within the session. In another embodiment of the invention, for treatment of severe cases of hyperhidrosis, the treatment regimen may comprise a series of longer sessions, or 10 to 20 second sessions spread over time, depending on the user's tolerance to electrical current at either the "HI" or "LO" setting. For those individuals who are particularly sensitive to electrical current the treatment session may be only at a "LO" setting. For those who can tolerate current well the session may be at a "HI" setting. Upon completion of a treatment session, the iontophoretic treatment device 10 is switched to the "0" position. A variable potentiometer may be substituted for the switch 24 to provide more precise current adjustment.

Through repeated use of the device the amount of aluminum ions available for penetration into the skin decreases. Eventually the amount decreases to a point where the device 10 is no longer effective. In accordance with the present invention, the applicator 20 may then be separated from the generator 39, as shown in FIG. 3, and a new applicator 20 installed in its place. In this sense, the applicator may be considered a disposable unit. It is also possible that the chemicals within the pads 22, 24 deplete before the aluminum electrodes 12, 14. In that case, a new set of pads 22, 24 may be installed in the applicator 20.

With the slow AC signal utilized in the system of the present invention, antiperspirant concentration can be increased substantially beyond the two percent level typically present in over-the-counter topical antiperspirants. With a positively charged aluminum ion antiperspirant carried by a pad 22, 24 when the positive half of the AC signal is driving the electrode 12, 14 associated with the pad, then the positive metallic ion component of the antiperspirant is repelled and driven into the skin. The sodium salicylate also contains a negative component, i.e. the salicylate ion. However, when the AC signal swings negative on the other half of the signal, the salicylate negative ion is driven into the skin. This enables substantially increased antiperspirant concentrations.

Since both electrodes are "active" with the simplified arrangement of the present invention, the device can deliver twice the amount of antiperspirant compared to a comparable DC iontophoretic device. For example, if the antiperspirant to be delivered is negative and the signal at one electrode 22, 24 is negative, then that pad delivers the antiperspirant to the skin. Simultaneously, the other electrode is positive and the same antiperspirant does not ordinarily flow.

Enhanced skin permeability occurs through the use of an appropriate salt of salicylic acid, such as sodium salicylate. The salicylate is electrically delivered into the treatment site and greatly lowers skin resistance and increases skin permeability thereby allowing for more effective iontophoretic treatment. In a preferred embodiment of the invention, the salicylate is carried, along with the antiperspirant chemicals, by the pads and is accordingly driven into the treatment region along with the antiperspirant chemicals.

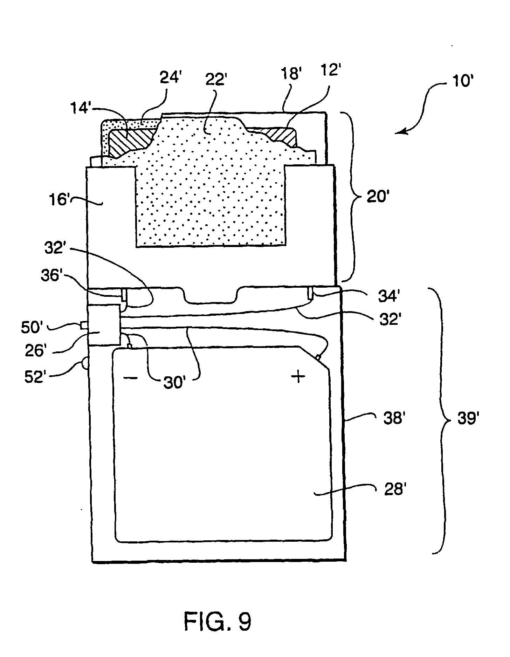

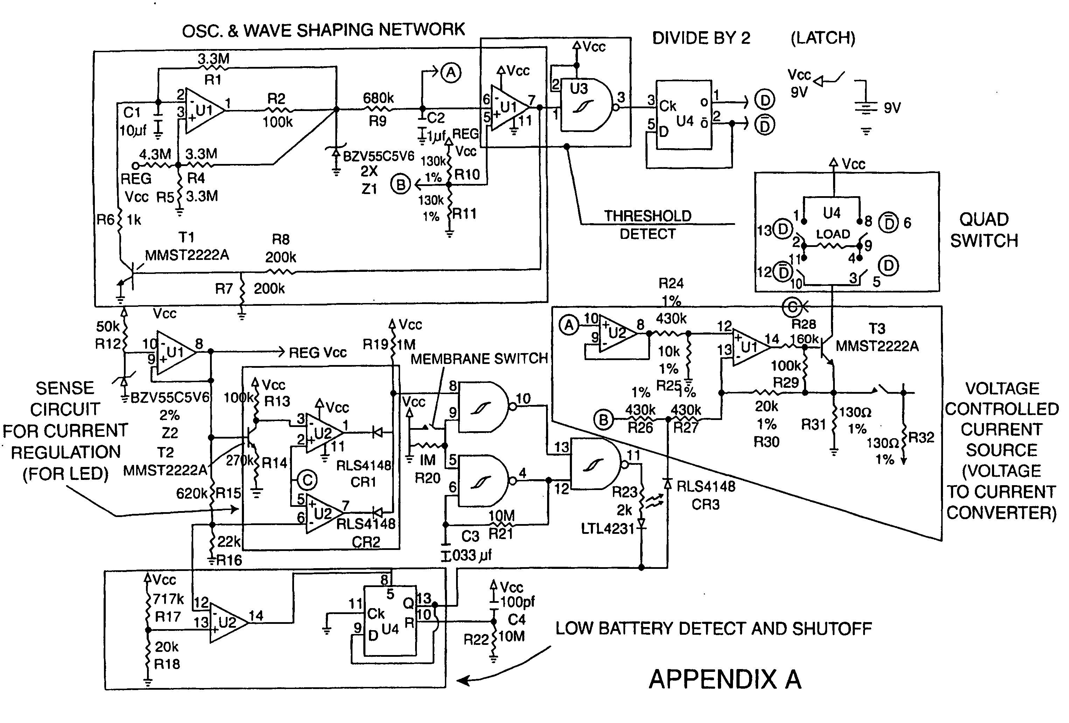

With reference to FIG. 9, there is shown a second configuration of a more compact iontophoretic device 10' which incorporates aspects of the present invention. Except for the size of the generator 39' and the battery power supply 28' included therein, this configuration of an iontophoretic device 10' is generally identical to the iontophoretic device 10' of FIGS. 2 through 5. For ease in correlating the two configurations, the numerals associated with elements of the second configuration are the same as those of the first configuration except that they are primed.

The second configuration of the iontophoretic device 10' is ideally suited for daily, short duration treatments, such as the 10 to 20 second treatment period previously described. The applicator 20' is identical to that of the first configuration device 10 (FIG. 2) and is removable from the generator 39' in the same manner as the first configuration. The battery power supply 28' (FIG. 9) comprises one 9V battery that drives the AC generator 26'. In a preferred embodiment of the second configuration, to maintain overall cost at a minimum, the frequency control present in the first configuration is absent, and an appropriate value of the frequency parameter is set during manufacture and prior to delivery to the ultimate user. With regard to frequency, the AC generator 26' is configured to ensure that at least one current reversal cycle occurs during the 10 to 20 second treatment period.

Hence, those concerned with development and use of hyperhidrosis treatment systems will appreciate that the present invention satisfies a long existing need in the art for a system capable of administering deep-penetrating iontophoretic application of antiperspirant chemicals to the human body in a short period of time, operates at a relatively low power rating and is conducive to daily use. Of course, it will be apparent that the sweat control system may be applied to any portion of the anatomy where sweating is a problem.

It will be apparent from the foregoing that, while particular forms of the invention have been illustrated and described, various modifications can be made without departing from the scope of the invention as defined by the appended claims.

## Claims

1. A sweat control system, comprising:
a source of electric current;
a controller;
electrode means connected to said controller; and
a preparation of sodium salicylate adjacent said electrode means and adapted to be delivered to a treatment site to enhance delivery of additional metallic ions to said treatment site.

2. The system of claim 1 wherein the preparation further includes an aluminum-based antiperspirant.

3. The system of claim 2 wherein the antiperspirant consists of one of either alumiuum-chlorohydrate or aluminum-zirconium.

4. The system of claim 1 wherein said electrode means are formed of sheet stock metal having an irregular, nonsmooth, surface.

5. The system of claim 1 wherein the sheet stock metal consists of aluminum, aluminum alloy, magnesium or magnesium alloy.

6. The system of claim 4 wherein said electrode means comprises sandblasted sheet stock metal.

7. The system of claim 4 wherein said electrode means comprises powdered metal formed on said sheet stock metal.

8. The system of claim 4 wherein said electrode means comprises aluminum oxide.

9. The system of claim 1, wherein said electrode means comprise:
a pair of electrodes mounted in generally close proximity to one another and separated by an insulating member, said electrodes carry an antiperspirant element and are responsive to said source of electrical current through said controller, one of said electrodes being connected to said source of electrical current and arranged to act primarily as a cathode, and the other of said electrodes being connected to said source of electrical current and arranged to act primarily as an anode; and the system further comprises
a pair of pads, each of said pads positioned in adjacent contact with one of said electrodes, each of said pads carrying sodium salicylate;
wherein said electrodes an sized and arranged so that the tissue to be treated can extend across said insulating member and simultaneously contact both of said pads.

10. The system of claim 9 wherein the pads further carry an aluminum-based antiperspirant.

11. The system of claim 10 wherein the antiperspirant consists of one of either aluminum-chlorohydrate or aluminum-zirconium.

12. The system of claim 9 wherein the electrodes are formed of sheet stock metal having an irregular, nonsmoothg, surface

13. The system of claim 9 wherein the sheet stock metal consists of aluminum, aluminum alloy, magnesium or magnesium alloy.

14. The system of claim 12, wherein the electrodes comprise sandblasted sheet stock metal.

15. The system of claim 12 wherein the electrodes comprise powdered metal formed an said sheet stock metal.

16. The system of claim 12 wherein the electrodes comprise aluminum oxide.

17. The system of claim 9, further comprising:
a controller for intermittently reversing, at a relatively low which prevents skin damage, between approximately 20 times per second and approximately once every three minutes, the polarity of said electrodes to cause said electrical current to flow in an opposite direction between said electrodes.

18. The system of claim 17 wherein the antiperspirant is aluminium based.

19. The system of claim 18 wherein the antiperspirant consists of one of either aluminum-chlorohydrate or aluminum-zirconium.

20. The system of claim 19 wherein the percentage of aluminumchlorohydrate or aluminum-zirconium contained within the antiperspirant is greater than 2%.

21. The system of claim 17 wherein the antiperspirant comprises an anticholinergic.

22. The system of any one of claims 17 through 21 wherein the pad further carries sodium salicylate.

23. The system of claim 1, wherein:
the source of electric current provides an AC waveform;
the electrode means comprise first and second electrodes mounted in generally close proximity to one another and separated by an tabulating member, said electrodes responsive to said AC waveform,
the system further comprising
a pair of pads, each of said pads positioned in adjacent contact with one of said electrodes, each of said pads carrying an antiperspirant, wherein said electrodes are sized and arranged so that the region to be treated can extend across said insulating member and simultaneously contact both of said pads.

24. The system of claim 23 wherein the first and second electrodes are responsive to the AC waveform such that the electrodes are of opposite polarities.

25. The system of claim 23 wherein the pads comprise a material containing the antiperspirant.

26. The system of claim 23 wherein the pads are formed of a fiber and the antiperspirant is saturated into the pads.

27. The system of claim 23 wherein tho antiperspirant comprises aluminum based chemicals.

28. The system of claim 21 wherein the antiperspirant comprises aluminum-chlorohydrate.

29. The system of claim 27 wherein the antiperspirant comprises aluminum-zirconium.

30. The system of claim 23 wherein the antiperspirant comprises an anticholinergic.

31. The system recited in any of claims 17-21 or 23-30, and further including with said antiperspirant a salt of salicylic acid.

32. The system of claim 23 wherein the electrodes are formed of sheet stock metal having an irregular, nonsmooth, surface area.

33. The system of claim 31 wherein the sheet stock metal consists of aluminum, aluminium alloy, magnesium, or magnesium alloy.

34. The system of claim 31, wherein the electrodes comprise sandblasted sheet stock metal.

35. The system of claim 31 wherein the electrodes comprise powdered metal formed on said sheet stock metal.

36. The system of claim 31 wherein the electrodes comprise aluminum oxide.

37. The system of claim 1, further comprising:
an iontophoretic delivery unit; wherein
the preparation of sodium salicylate is adapted for delivery to a subject by said iontophoretic delivery unit.

38. A system as recited in claim 37 wherein said iontophoretic delivery unit provides a low frequency A.C. signal to deliver said preparation.

39. A system as set forth in claim 38 wherein said A.C. signal is in the range of 10 Hz-0.0027 Hz.

40. A system as recited in any one of claims 37-39 wherein said preparation further includes an anticholinergic substance.

41. A system as recited in any one of claims 37-39 wherein said preparation further includes aluminum chlorohydrate.

42. A system as recited in any one of claims 37-39 wherein said preparation further includes aluminum-zirconium.

## Patentansprüche

1. System zur Schweißregelung, wobei das System folgendes umfasst:
eine Quelle für elektrischen Strom;
eine Regelungseinrichtung;
mit der genannten Regelungseinrichtung verbundene Elektrodeneinrichtungen; und
eine Präparation aus Natriumsalicylat angrenzend an die genannten Elektrodeneinrichtungen, und wobei die Präparation in der Lage ist, einem Behandlungsort zugeführt zu werden, um die Zufuhr zusätzlicher Metallionen an den genannten Behandlungsort zu fördern.

2. System mach Anspruch 1, wobei die Präparation ferner ein Antitranspirant auf Aluminiumbasis aufweist.

3. System nach Anspruch 2, wobei das Antitranspirant aus Alumium-Chlorhydrat oder Aluminium-Zirkonium besteht.

4. System nach Anspruch 1, wobei die genannten Elektrodeneinrichtungen aus Metallblech mit einer unregelmäßigen, unebenen Oberfläche gebildet werden.

5. System nach Anspruch 1, wobei das Metallblech aus Aluminium, Aluminiumlegierung, Magnesium oder Magnesiumlegierung besteht.

6. System nach Anspruch 4, wobei die genannten Elektrodeneinrichtungen sandgestrahltes Metallblech umfassen.

7. System nach Anspruch 4, wobei die genannten Elektrodeneinrichtungen pulverförmiges Metall umfassen, das an dem genannten Metallblech ausgebildet ist.

8. System nach Anspruch 4, wobei die genannten Elektrodeneinrichtungen Aluminiumoxid umfassen.

9. System nach Anspruch 1, wobei die genannten Elektrodeneinrichtungen folgendes umfassen:
ein Paar von Elektroden, die allgemein sehr nah aneinander angebracht sind und durch ein isolierendes Element getrennt sind, wobei die genannten Elektroden ein Antitranspirantelement aufweisen und auf die genannte Quelle für elektrischen Strom über die Regelungseinrichtung ansprechen, wobei eine der genannten Elektroden mit der genannten Quelle für elektrischen Strom verbunden und so angeordnet ist, dass sie primär als Kathode fungiert, und wobei die andere der genannten Elektroden mit der genannten Quelle für elektrischen Strom verbunden und so angeordnet ist, dass sie primär als Anode fungiert, und wobei das System ferner folgendes umfasst:
ein Paar von Polstern, wobei jedes der genannten Polster in angrenzendem Kontakt mit einer der genannten Elektroden positioniert ist, wobei jedes der genannten Polster Natriumsalicylat trägt;
wobei die genannten Elektroden so bemessen und angeordnet sind, dass sich das zu behandelnde Gewebe über das genannte isolierende Element erstrecken und gleichzeitig beide genannten Polster berühren kann.

10. System nach Anspruch 9, wobei die Polster ferner ein Antitranspirant auf Aluminiumbasis tragen.

11. System nach Anspruch 10, wobei das Antitranspirant aus Aluminium-Chlorhydrat oder Aluminium-Zirkonium besteht.

12. System nach Anspruch 9, wobei die Elektroden aus Metallblech mit einer unregelmäßigen, unebenen Oberfläche gebildet werden.

13. System nach Anspruch 9, wobei das Metallblech aus Aluminium, Aluminiumlegierung, Magnesium oder Magnesiumlegierung besteht.

14. System nach Anspruch 12, wobei die Elektroden sandgestrahltes Metallblech umfassen.

15. System nach Anspruch 12, wobei die Elektroden an dem genannten Metallblech ausgebildetes pulverförmiges Metall umfassen.

16. System nach Anspruch 12, wobei die Elektroden Aluminiumoxid umfassen.

17. System nach Anspruch 9, wobei dieses ferner folgendes umfasst:
eine Regelungseinrichtung zur absatzweisen Umkehr der Polarität der genannten Elektroden mit einer verhältnismäßig niedrigen Frequenz, welche Hautverletzungen verhindert, zwischen ungefähr 20 Mal pro Sekunde und ungefähr alle drei Minuten, um zu bewirken, dass der genannte elektrische Strom zwischen den genannten Elektroden in eine entgegengesetzte Richtung fließt.

18. System nach Anspruch 17, wobei das Antitranspirant auf Aluminium basiert.

19. System nach Anspruch 18, wobei das Antitranspirant aus Aluminium-Chlorhydrat oder Aluminium-Zirkonium besteht.

20. System nach Anspruch 19, wobei der prozentuale Anteil von Aluminium-Chlorhydrat oder Aluminium-Zirkonium in dem Antitranspirant größer ist als 2 %.

21. System nach Anspruch 17, wobei das Antitranspirant ein Anticholinergika umfasst.

22. System nach einem der Ansprüche 17 bis 21, wobei die Polster ferner Natriumsalicylat tragen.

23. System nach Anspruch 1, wobei:
die Quelle für elektrischen Strom eine Wechselstrom-Kurvenform bereitstellt;
die Elektrodeneinrichtungen erste und zweite Elektroden umfassen, die allgemein sehr nah aneinander angebracht und durch ein isolierendes Element voneinander getrennt sind,
wobei die genannten Elektroden auf die genannte Wechselstrom-Kurvenform ansprechen;
wobei das System ferner folgendes umfasst:
ein Paar von Polstern, wobei jedes der genannten Polster in angrenzendem Kontakt mit einer der genannten Elektroden positioniert ist, wobei jedes der genannten Polster ein Antitranspirant trägt, wobei die genannten Elektroden so bemessen und angeordnet sind, dass sich der zu behandelnde Bereich über das genannte isolierende Element erstrecken und gleichzeitig beide der genannten Polster berühren kann.

24. System nach Anspruch 23, wobei die ersten und zweiten Elektroden auf die Wechselstrom-Kurvenform so ansprechen, dass die Elektroden entgegengesetzte Polaritäten aufweisen.

25. System nach Anspruch 23, wobei die Polster ein Material umfassen, das das Antitranspirant aufweist.

26. System nach Anspruch 23, wobei die Polster aus einer Faser gebildet werden, und wobei das Antitranspirant in den Polstern gesättigt ist.

27. System nach Anspruch 23, wobei das Antitranspirant Chemikalien auf Aluminiumbasis umfasst.

28. System nach Anspruch 27, wobei das Antitranspirant Aluminium-Chlorhydrat umfasst.

29. System nach Anspruch 27, wobei das Antitranspirant Aluminium-Zirkonium umfasst.

30. System nach Anspruch 23, wobei das Antitranspirant ein Anticholinergika umfasst.

31. System nach einem der Ansprüche 17 bis 21 oder 23 bis 30, wobei es ferner in Verbindung mit dem genannten Antitranspirant ein Salz aus Salicylsäure aufweist.

32. System nach Anspruch 23, wobei die Elektroden aus Metallblech mit einer unregelmäßigen, unebenen Oberfläche gebildet werden.

33. System nach Anspruch 31, wobei das Metallblech aus Aluminium, Aluminiumlegierung, Magnesium oder Magnesiumlegierung besteht.

34. System nach Anspruch 31, wobei die Elektrodeneinrichtungen sandgestrahltes Metallblech umfassen.

35. System nach Anspruch 31, wobei die Elektroden an dem genannten Metallblech ausgebildetes pulverförmiges Metall umfassen.

36. System nach Anspruch 31, wobei die Elektroden Aluminiumoxid umfassen.

37. System nach Anspruch 1, wobei dieses ferner folgendes umfasst:
eine iontophoretische Verabreichungseinheit; wobei
die Präparation aus Natriumsalicylat sich für die Verabreichung an einen Patienten durch die genannte iontophoretische Verabreichungseinheit eignet.

38. System nach Anspruch 37, wobei die genannte iontophoretische Verabreichungseinheit ein Niederfrequenz-Wechselstromsignal für die Verabreichung der genannten Präparation bereitstellt.

39. System nach Anspruch 38, wobei das genannte Wechselstromsignal im Bereich von 10 Hz bis 0,0027 Hz liegt.

40. System nach einem der Ansprüche 37 bis 39, wobei die genannte Präparation ferner eine anticholinergische Substanz aufweist.

41. System nach einem der Ansprüche 37 bis 39, wobei die genannte Präparation ferner Aluminium-Chlorhydrat aufweist.

42. System nach einem der Ansprüche 37 bis 39, wobei die genannte Präparation ferner Aluminium-Zirkonium aufweist.

## Revendications

1. Système de contrôle de la transpiration, comprenant:
une source de courant électrique;
un dispositif de commande;
des moyens formant électrode reliés au dit dispositif de commande; et
une préparation de salicylate de sodium adjacente aux dits moyens formant électrode et adaptée pour être fournie à un site de traitement pour optimiser la remise d'ions métalliques supplémentaires audit site de traitement.

2. Système selon la revendication 1, dans lequel la préparation comprend en outre un anti-transpirant à base d'aluminium.

3. Système selon la revendication 2, dans lequel l'anti-transpirant est constitué d'un parmi le chlorhydrate d'aluminium et le zirconium d'aluminium.

4. Système selon la revendication 1 dans lequel lesdits moyens formant électrode sont formés d'un métal en feuille présentant une surface irrégulière non lisse.

5. Système selon la revendication 1, dans lequel le métal en feuille est constitué d'aluminium, d'un alliage d'aluminium, de magnésium ou d'un alliage de magnésium.

6. Système selon la revendication 4, dans lequel lesdits moyens formant électrode comprennent un métal en feuille sablé.

7. Système selon la revendication 4, dans lequel lesdits moyens formant électrode comprennent un métal en poudre formé sur ledit métal en feuille.

8. Système selon la revendication 4, dans lequel lesdits moyens formant électrode comprennent de l'oxyde d'aluminium.

9. Système selon la revendication 1, dans lequel lesdits moyens formant électrode comprennent:
une paire d'électrodes montées à proximité généralement étroite l'une de l'autre et séparées par un élément isolant, lesdites électrodes transportent un élément anti-transpirant et sont réactives à ladite source de courant électrique à travers ledit dispositif de commande, une desdites électrodes étant raccordée à ladite source de courant électrique et agencée pour agir principalement comme une cathode, et l'autre desdits électrodes étant raccordée à ladite source de courant électrique et agencée pour agir principalement comme une anode; et le système comprend en outre
une paire de tampons, chacun desdits tampons positionnés en contact adjacent avec une desdites électrodes, chacun desdits tampons transportant du salicylate de sodium;
dans lequel lesdites électrodes sont dimensionnées et agencées de sorte que le tissu à tester peut s'étendre à travers ledit élément isolant et entrer simultanément en contact avec les deux tampons.

10. Système selon la revendication 9, dans lequel les tampons transportent en outre un anti-transpirant à base d'aluminium.

11. Système selon la revendication 10, dans lequel l'anti-transpirant est constitué d'un parmi le chlorhydrate d'aluminium et le zirconium d'aluminium.

12. Système selon la revendication 9, dans lequel les électrodes sont formées d'un métal en feuille présentant une surface irrégulière non lisse.

13. Système selon la revendication 9, dans lequel le métal en feuille est constitué d'aluminium, d'un alliage d'aluminium, de magnésium ou d'un alliage de magnésium.

14. Système selon la revendication 12, dans lequel les électrodes comprennent un métal en feuille sablé.

15. Système selon la revendication 12, dans lequel les électrodes comprennent un métal en poudre formé sur ledit métal en feuille.

16. Système selon la revendication 12, dans lequel les électrodes comprennent de l'oxyde d'aluminium.

17. Système selon la revendication 9, comprenant en outre:
un dispositif de commande destiné à inverser de façon intermittente, à une fréquence relativement basse qui empêche l'endommagement de la peau, entre environ 20 fois par seconde et environ une fois toutes les trois minutes, la polarité desdites électrodes pour amener ledit courant électrique à circuler dans un sens opposé entre lesdites électrodes.

18. Système selon la revendication 17, dans lequel l'anti-transpirant est à base d'aluminium.

19. Système selon la revendication 18, dans lequel l'anti-transpirant est constitué d'un parmi le chlorhydrate d'aluminium et le zirconium d'aluminium.

20. Système selon la revendication 19, dans lequel le pourcentage de chlorhydrate d'aluminium ou de zirconium d'aluminium contenu dans l'anti-transpirant est supérieur à 2 %.

21. Système selon la revendication 17, dans lequel l'anti-transpirant comprend un anticholinergique.

22. Système selon l'une quelconque des revendications 17 à 21, dans lequel le tampon transporte en outre du salicylate de sodium.

23. Système selon la revendication 1, dans lequel:
la source de courant électrique fournit une forme d'onde de courant alternatif;
les moyens formant électrode comprennent des première et deuxième électrodes montées à proximité généralement étroite l'une de l'autre et séparées par un élément isolant, lesdites électrodes réactives à ladite forme d'onde de courant alternatif;
le système comprenant en outre
une paire de tampons, chacun desdits tampons positionnés en contact adjacent avec une desdites électrodes, chacun desdits tampons transportant un anti-transpirant, dans lequel lesdites électrodes dimensionnées et agencées de sorte que la région à traiter peut s'étendre à travers ledit élément isolant et entrer simultanément en contact avec les deux tampons.

24. Système selon la revendication 23, dans lequel les première et deuxième électrodes sont réactives à la forme d'onde de courant alternatif de sorte que les électrodes sont de polarités opposées.

25. Système selon la revendication 23, dans lequel les tampons comprennent un matériau contenant l'anti-transpirant.

26. Système selon la revendication 23, dans lequel les tampons sont formés d'une fibre et l'anti-transpirant est saturé dans les tampons.

27. Système selon la revendication 23, dans lequel l'anti-transpirant comprend des produits chimiques à base d'aluminium.

28. Système selon la revendication 27, dans lequel l'anti-transpirant comprend du chlorhydrate d'aluminium.

29. Système selon la revendication 27, dans lequel l'anti-transpirant comprend du zirconium d'aluminium.

30. Système selon la revendication 23, dans lequel l'anti-transpirant comprend un anticholinergique.

31. Système selon l'une quelconque des revendications 17 à 21 ou 23 à 30, et comprenant en outre avec ledit anti-transpirant un sel d'acide salicylique.

32. Système selon la revendication 23, dans lequel les électrodes sont formées de métal en feuille ayant une zone de surface irrégulière, non lisse.

33. Système selon la revendication 31, dans lequel le métal en feuille est constitué d'aluminium, d'un alliage d'aluminium, de magnésium ou d'un alliage de magnésium.

34. Système selon la revendication 31, dans lequel les électrodes comprennent un métal en feuille sablé.

35. Système selon la revendication 31, dans lequel les électrodes comprennent un métal en poudre formé sur ledit métal en feuille.

36. Système selon la revendication 31, dans lequel les électrodes comprennent de l'oxyde d'aluminium.

37. Système selon la revendication 1, comprenant en outre:
une unité de remise iontophorétique; dans lequel
la préparation de salicylate de sodium est adaptée pour être remise à un sujet par ladite unité de remise iontophorétique.

38. Système selon la revendication 37, dans lequel ladite unité de remise iontophorétique fournit un signal de courant alternatif à basse fréquence pour remettre ladite préparation.

39. Système selon la revendication 38 dans lequel ledit signal de courant alternatif est dans la plage de 10 Hz à 0,0027 Hz.

40. Système selon l'une quelconque des revendications 37 à 39, dans lequel ladite préparation comprend en outre une substance anticholinergique.

41. Système selon l'une quelconque des revendications 37 à 39, dans lequel ladite préparation comprend en outre du chlorhydrate d'aluminium.

42. Système selon l'une quelconque des revendications 37 à 39, dans lequel ladite préparation comprend en outre du zirconium d'aluminium.
